# EUROPEAN PATENT APPLICATION

(11) **EP 4 389 804 A1**
(43) Date of publication of application: **26.06.2024**
(21) Application number: 22858791.1
(22) Date of filing: 19.08.2022
(51) Int. Cl.: C08J 3/24, C08J 3/075, C08J 3/12, C08B 37/08, C08K 3/20

(54) **METHOD FOR PREPARING SWELLABLE POLYMER TO WHICH OBJECT IS FIXED**

(30) Priority: 20.08.2021 KR 20210109892; 10.08.2022 KR 20220100043
(71) Applicant: Pusan National University Industry-University Cooperation Foundation, Busan 46241 (KR)
(72) Inventor: YANG, Seung Yun, Miryang-si Gyeongsangnam-do 50463 (KR); KIM, Sodam, Busan 49126 (KR); LEE, Jian, Busan 48045 (KR); KIM, Se Min, Gimhae-si Gyeongsangnam-do 51023 (KR)
(74) Representative: Schmid, Nils T.F.
(86) International application number: PCT/KR2022/012398
(87) International publication number: WO 2023/022554

(57) **Abstract**

The present invention provides a method for preparing a swellable polymer to which an object is fixed. The preparation method of the present invention comprises a step of absorbing a solution of an object, which is dissolved or dispersed in a solvent with affinity for a swellable polymer, into a dried swellable polymer comprising a linker having a functional group that can bind to the object. Unlike a conventional preparation method in which an object is fixed before a swellable polymer is cross-linked, a method using a swellable polymer so as to fix an object after a polymer is cross-linked is used, and thus an object can be more rapidly fixed with efficiency higher than that of a conventional technique.

## Description

### FIELD

The present disclosure relates to a method for preparing a swellable polymer having an object immobilized thereto, more specifically, to a method for immobilizing an object to a swellable polymer using absorption ability of a dried swellable polymer.

### DESCRIPTION OF RELATED ART

A swellable polymer is a polymer that absorbs a solvent and swells based on absorbent properties of the polymer, and is useful in a wide range of fields such as medicine, agriculture, and biotechnology. In general, the swellable polymer is used as a carrier to deliver a target substance by carrying the target substance in a space between cross-links of the polymer using swelling behavior of the swellable polymer. In this regard, a reaction step is performed to ensure a stable bond between the swellable polymer and the target substance.

Currently, this step is performed before cross-linking of the swellable polymer. That is, the polymer and the target substance first react with and is bonded to each other, and then the swellable polymer is cross-linked. However, this scheme is time-consuming. Further, although a large amount of a immobilizing material is used, efficiency of immobilizing the target substance is not good. Therefore, a method for quickly bonding a large amount of the target substance thereto per unit volume of the swellable polymer is needed.

In one example, although the mortality rate of cancer disease is decreasing due to early diagnosis and the development of medical technology, the caner is still the number one cause of death worldwide. In clinical practice, cancer treatment methods are broadly classified into three types: surgical therapy and radiation therapy to remove cancer locally, and chemotherapy using anticancer drugs when cancer has spread throughout the body. In particular, the radiation therapy is widely used in more than 50% of cancer patients either alone or in combination with chemical treatment.

Among radiation treatment schemes, therapy using radioisotopes does not require large equipment and has the advantage of minimizing patient pain because radioisotopes is administered using a syringe more easily unlike existing external radiation therapy. Radioisotopes currently being applied include ⁹⁰Y, ⁶⁷Cu, ¹⁸⁸Re, ¹⁷⁷Lu, and ¹³¹I, and are injected into the body in a solution phase through intravenous injection. However, radioactive isotopes circulating in the body cause damage to normal cells and tissues due to reduced selectivity to tumor cells, and cause side effects such as abnormal hormone secretion and hair loss in patients.

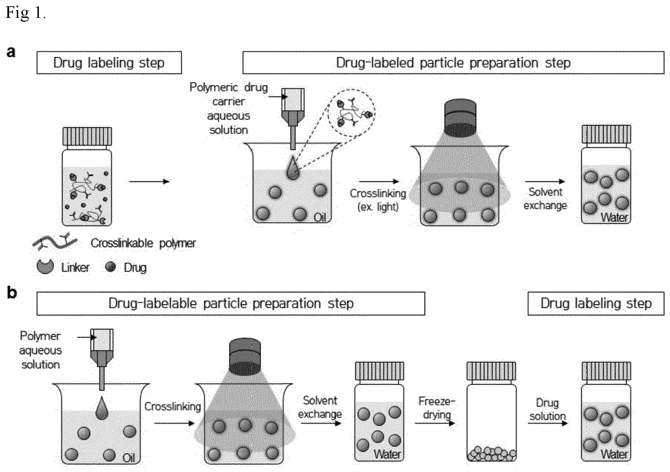

To solve this problem, a technology has been recently developed in which the radioactive isotopes are bonded (labeled) to biodegradable polymers via a chemical reaction in a solution phase, and a polymer-radioisotope (drug) conjugate prepared in this way is formulated into microparticles that are injectable and may remain in the body for a long time, thereby allowing radiation treatment only to local areas (a in FIG. 1). However, when the polymer-radioactive drug conjugate with a half-life is prepared and is formulated into the microparticles for injection into the body, multiple steps and a long time are required. Thus, it is difficult to prepare the same immediately at the hospital site, and the long formulation process results in the loss of radioactive drugs and a decrease in radioactivity.

### DISCLOSURE

### TECHNICAL PURPOSE

The present disclosure is provided in response to the above-mentioned problems and needs. Thus, a purpose of the present disclosure is to provide a new method for quickly and easily bonding a large amount of a target substance to a well-swellable polymer.

A purpose of the present disclosure is to provide a method for preparing a swellable polymer having an object immobilized thereto in which the object may be immobilized to the swellable polymer at high efficiency using the absorption ability of dried swellable polymer.

### TECHNICAL SOLUTION

The present disclosure provides a method for preparing a swellable polymer having an object immobilized thereto, the method comprising: absorbing an object solution into a dried swellable polymer, wherein the dried swellable polymer contains a linker having a functional group capable of binding to an object, wherein the object solution includes a solvent having affinity to the swellable polymer, and the object dissolved or dispersed in the solvent.

As used herein, the object is a word that has the same meaning as an object to be immobilized, and refers to an object that is immobilized to the swellable polymer. For example, the object may be a drug for treatment or prevention in the body. In the present disclosure, the swellable polymer refers to a polymer that is crosslinked and does not dissolve in a solvent, but quickly absorbs a solvent having affinity thereto and expands its volume. For example, an example of the swellable polymer that absorbs a hydrophilic solvent and does not dissolve in the hydrophilic solvent may include a hydrogel such as polysaccharides such as hyaluronic acid, alginate, and chitosan, polyethylene glycol (PEG), polyvinyl alcohol (PVA), gelatin, collagen, and albumin hydrogel. An example of the swellable polymer that absorbs a hydrophobic solvent and does not dissolve in a hydrophobic organic solvent may include a crosslinked polymer of PLA-diacrylate, PGA-diacrylate, or PLGA-diacrylate including polylactide, polyglycolide, or poly(lactide-co-glycolide) (PLGA) as a copolymer thereof as a hydrophobic polymer, and including methacarylate (MA) as a photocrosslinking group. Preferably, the swellable polymer of the present disclosure may be a biocompatible polymer.

Conventionally, in preparing a compound in which the object is bonded to the swellable polymer, the object is first immobilized to the polymer, and then the polymer is cross-linked. However, the present disclosure provides a method of first crosslinking the polymer and then immobilizing the object thereto. The inventor of the present disclosure has newly discovered that when a cross-linked and dried swellable polymer containing a functional group or linker that can bind to the object is prepared, and then, a solution in which the object is dissolved or dispersed in a solvent that has affinity to the swellable polymer is absorbed into the cross-linked and dried swellable polymer, a high binding efficiency may be achieved even with a small amount of the object solution. Moreover, the inventor of the present disclosure has newly discovered that the method for preparing the present disclosure is not intended to prepare the polymer to which the object is pre-immobilized, but is able to prepare very easily the polymer having the object immobilized thereto by reacting the dried swellable polymer with the solution containing an object at a site requiring the product.

In accordance with the present disclosure, the object may be any one selected from the group consisting of radioactive isotopes, metals, ionic compounds, and organic compounds.

Preferably, the object may be a substance that provides a pharmacological effect in vivo.

The radioactive isotope may be one or more selected from radioactive halogen elements or radioactive metal elements, For example, the radioactive isotope may be any one or more selected from the group consisting of ¹²³I, ¹²⁴I, ¹²⁵I, ¹³¹I, ²¹¹At, ⁶⁴Cu, ⁶⁷Ga, ⁶⁸Ga, ⁴⁴Sc, ⁴⁷Sc, ¹¹¹In, ¹⁷⁷Lu, ⁸⁶Y, ⁹⁰Y, ⁸⁹Zr, ²¹²Bi, ²¹³Bi, ¹⁶⁶Ho, ^{99m}Tc ²¹²Pb and ²²⁵Ac.

The metal may be any one or more selected from the group consisting of gold (Au), platinum (Pt), silver (Ag), aluminum (Al), copper (Cu), nickel (Ni), cobalt (Co), palladium (Pd), chromium (Cr), and manganese (Mn), and alloys thereof. However, the present disclosure does not necessarily limit the types of the radioisotopes and the metals to those as described above.

Abond between the object and the linker is not limited particularly as long as the object can be immobilized to the swellable polymer. For example, the bond may a covalent bond, a hydrogen bond, a coordinate bond, a non-covalent bond such as an ionic bond or a van der Waals bond. However, the present disclosure is not limited to this specific bond. In particular, any bond may available as long as when the swellable polymer is injected into the living body to perform a desired function, the object is immobilized to the polymer via the bond.

In one embodiment of the present disclosure, when the object is a metal or ionic compound, the linker may be a ligand compound capable of having a coordinate bond to the metal or ionic compound.

In one embodiment of the present disclosure, when the object has a cationic functional group, the ligand may be a chelator. For example, the swellable polymer to which the object is immobilized via a bond between a cationic metal and a chelator may have a structure of each of following Chemical Formulas 1 to 3:

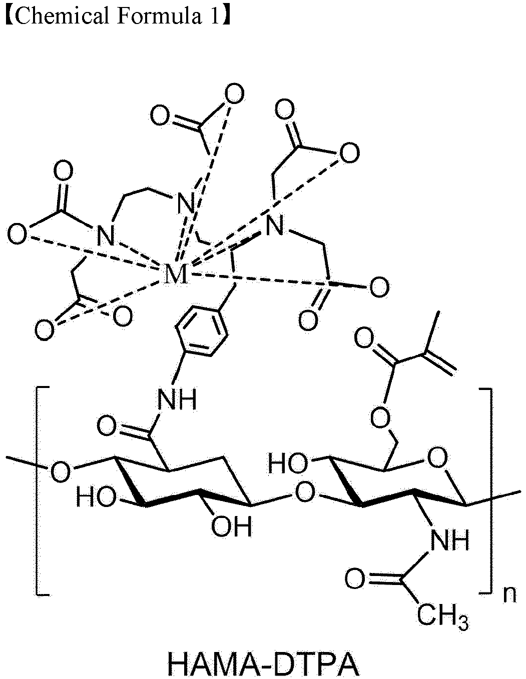

In the Chemical Formula 1, M may be a radioactive metal element, and n may be an integer of 1 or greater.

In the swellable polymer having the structure of the Chemical Formula 1, the M may be specifically selected from the group of ⁶⁴Cu, ⁶⁷Ga, ⁶⁸Ga, ⁴⁴Sc, ⁴⁷Sc, ¹¹¹In, ¹⁷⁷Lu, ⁸⁶Y, ⁹⁰Y, ⁸⁹Zr, ²¹²Bi, ²¹³Bi, ¹⁶⁶Ho and ^{99m}Tc. The M may have the coordinate bond to and be labeled to diethylenetriaminepentaacetic acid (DTPA) binding to hyaluronic acid methacrylate (HAMA) of the Chemical Formula 1.

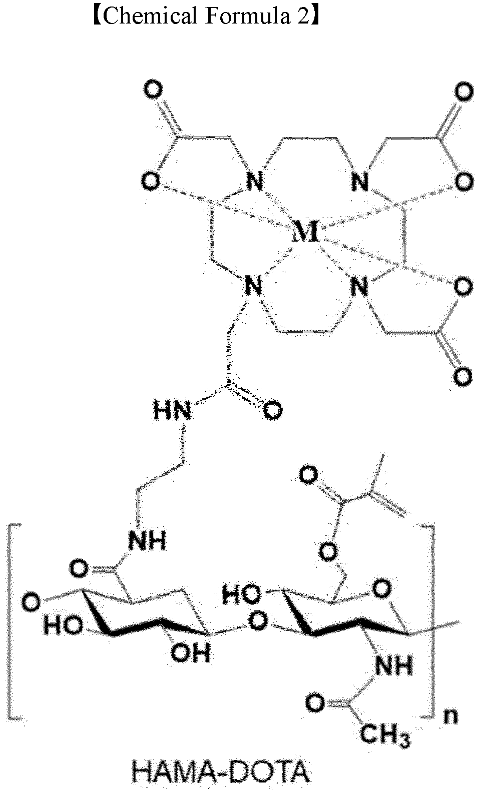

In the Chemical Formula 2, M may be a radioactive metal element, and n may be an integer of 1 or greater.

In the swellable polymer having the structure of the Chemical Formula 2, the M may be specifically selected from the group consisting of ⁶⁴Cu, ⁶⁷Ga, ⁶⁸Ga, ⁴⁴Sc, ⁴⁷Sc, ¹¹¹In, ¹⁷⁷Lu, ⁸⁶Y, ⁹⁰Y, ⁸⁹Zr, ²¹³Bi, ²¹²Pb and ²²⁵Ac. The M may have the coordinate bond to and be labeled to to dodecane tetraacetic acid (DOTA) bound to hyaluronic acid methacrylate (HAMA) of the Chemical Formula 2.

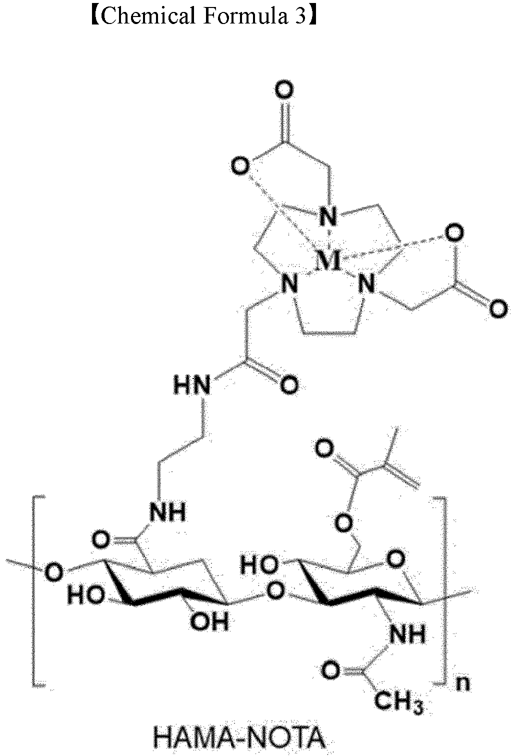

In the Chemical Formula 3, M may be a radioactive metal element, and n may be an integer of 1 or greater.

In the swellable polymer having the structure of the Chemical Formula 3, the M may be specifically selected from the group consisting of ⁶⁴Cu, ⁶⁷Ga, ⁶⁸Ga, ⁴⁴Sc, ⁴⁷Sc, ¹¹¹In, ¹⁷⁷Lu, ⁸⁶Y, ⁹⁰Y, ²¹²Bi, ²¹³Bi and ²¹²Pb. The M may have the coordinate bond to and be labeled to triazacyclononane triacetic acid (NOTA) bound to hyaluronic acid methacrylate (HAMA) of the Chemical Formula 3.

According to the method for preparing the swellable polymer having the object immobilized thereto of claim, the chelator may be selected from a group consisting of DOTA, DOTA derivatives, DOTA-GA, DO2A, HBED-CC, NOTA, p-SCN-Bn-NOTA, NODA-GA, PrP9, DTPA, CHX-A"-DTPA-isothiocyanate, CHX-A"-DTPA-maleimide, CHX-A"-DTPA-glutaric acid NHS ester, DTPA derivatives (1M3B-DTPA, 1B3M-DTPA, 1B4M-DTPA, CHX-A-DTPA and CHX-B-DTPA), NETA, NE3TA, NE3TA-Bn, C-NETA, C-NE3TA, C-DOTA-isothiocyanates, MeO-DOTA-isothiocyanates, PA-DOTA-isothiocyanates, 1B4M-DTPA-isothiocyanates, 2-(4-nitrobenzyl)-cyclen, 2-(4-nitrobenzyl)-cyclam, 2-(4-benzamidobenzyl)-NOTA, 2-(4-nitrobenzyl)-DOTA, 2-(4-nitrobenzyl)-TETA, 1-(4-nitro)-B4NMTPA, TETA, TE2A, TE2A derivatives (CB-TE2A, CB-TE1A1P, CB-TE2P, MM-TE2A, DM-TE2A), cyclam, cyclam derivatives (1-(4-nitrobenzyl)-cyclam, 6-(4-nitrobenzyl)-cyclam), CB-TE2A, CB-TE2A derivatives (3,3'-(1,4,8,11-tetraazabicyclo[6.6.2]hexadecane-4,11-diyl)dipropanoic acid), CB-DO2A, CB-DO2A derivatives (3,3'-(1,4,7,10-tetraazabicyclo[5.5.2]tetradecane-4,10-diyl)dipropanoic acid), DiAmSar, SarAr, AmBaSar, DiAmsar derivatives, AAZTA, NOPO and TACN-TM. However, the present disclosure is not necessarily limited to these types.

In one embodiment, when the object has an anionic functional group, the functional group of the linker may be an aromatic compound or a heterocyclic compound, and the anionic functional group and the functional group of the linker may bind to each other via an electrophilic substitution reaction. For example, the heterocyclic compound is selected from a group consisting of imidazole, pyrrole, furan, thiophene, indole, and 3,4-dihydroxyphenyl.

In one example, the swellable polymer having the object immobilized thereto via a bond between the anionic functional group and the chelator may have a structure of a following Chemical Formula 4:

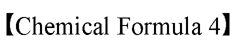

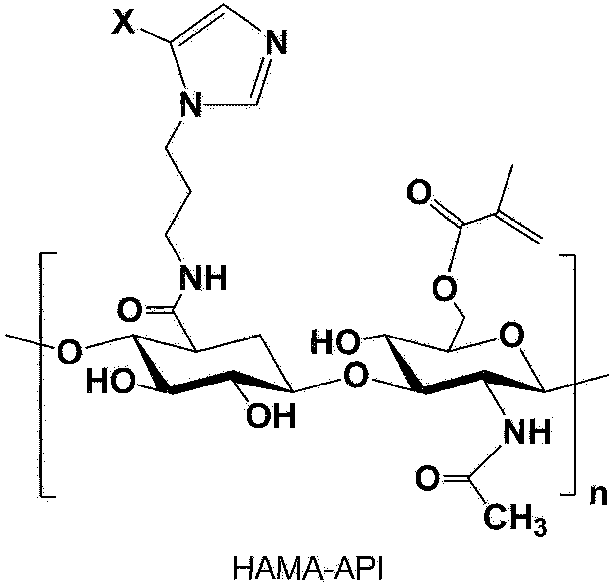

In the Chemical Formula 4, X may be a radioactive halogen element, and n may be an integer of 1 or greater. The X may be labeled to aminopropyl imidazole (API) bound to hyaluronic acid methacrylate (HAMA) of the Chemical Formula 4.

In one embodiment, when the object has a functional group that can be ionized, and the functional group has an opposite charge to a charge of the cross-linked swellable polymer, the object may be immobilized thereto via an ionic bond. For example, when the object has a cationic amine group (-NH₂), the object may be immobilized to the HMMA via an ionic bond to a carboxyl group (-COOH) as the anion of HAMA. Conversely, when the object has an anionic -COOH or sulfonic acid group (-SO₃H), an amine group may be introduced directly into HAMA via a diacetyl reaction, or a linker with an amine group may be introduced to HMMA to fix the object to HMMA via an ionic bond. In particular, a protein composed of amino acids may be easily immobilized to HMMA because the amino acid has -COOH and - NH₂ at both opposing ends.

In one embodiment, the object may be an organic compound, and the organic compound may include at least one first functional group selected among an amine group, a carboxyl group, a hydroxyl group, and a thiol group, and the linker may have a second functional group binding to the first functional group.

In one embodiment, when the organic compound includes an amine group (-NH₂), the functional group of the linker may include an aldehyde group (-CHO) that can bind to the amine group. The amine group and the aldehyde group react with each other and bind to each other such that the object may be immobilized to the swellable polymer.

In another embodiment, when the functional group of the organic compound includes a carboxyl group (-COOH), the functional group of the linker may include an amine group (-NH₂) that can bind to the carboxyl group. The carboxyl group and the amine group react with each other and bind to each other such that the object may be immobilized to the swellable polymer.

In still another embodiment, when the functional group of the organic compound includes a hydroxy group (-OH), the functional group of the linker may include a carboxyl group (-COOH). The hydroxyl group and the carboxyl group react with each other and bind to each other such that the object may be immobilized to the swellable polymer.

In still yet another embodiment, when the functional group of the organic compound includes a thiol group (-SH), the functional group of the linker may include a disulfide group (-S-S-). The thiol group and the disulfide group react with each other and bind to each other such that the object may be immobilized to the swellable polymer.

In one example, according to the present disclosure, the dried swellable polymer may have been prepared through any method selected from freeze drying, hot air drying, atmospheric drying, and vacuum drying. Preferably, the drying may the freeze-drying. Moreover, the dried swellable polymer may be in a powder or aerogel form.

Before performing the step of absorbing the object solution into the dried swellable polymer, an additional step may be performed to react a binder that has a functional group capable of binding to the object and binding to the linker of the dried swellable polymer therewith. However, the present disclosure is not necessarily limited thereto, and this step may be performed selectively when necessary.

The step of absorbing the object solution into the dried swellable polymer may be performed by immersing the dried swellable polymer in the object solution. Preferably, the object solution may be slowly added dropwise or added to the dried swellable polymer to provide a sufficient time for the dried swellable polymer to absorb the object solution. Through the absorption step, the object may be immobilized to the swellable polymer by chemically bonding to the linker instead of being carried in the swellable polymer.

### TECHNICAL EFFECTS

The dried swellable polymer has a porous structure and has the property of quickly absorbing the object solution through osmosis. This increases a probability at which the quickly absorbed object solution rapidly encounters the functional group of the swellable polymer, thereby providing highly efficient binding using a small amount of the object solution.

Moreover, according to the present disclosure, the swellable polymer may be labeled with the object within minutes at a site that requires the swellable polymer having the object immobilized thereto (e.g., a hospital). In a single process, the object may be labeled to the polymer at high efficiency, thereby improving user convenience and dramatically reducing object waste. Moreover, when using the radioactive isotope as the object, the polymer may be labeled with the object on-site in a short period of time, thereby minimizing the decrease in activity of the radioisotope.

Moreover, the method for preparing the swellable polymer having the object immobilized thereto as proposed in the present disclosure is not limited to labeling of one substance, but may be applied to labeling of various types of drugs, from organic and low-molecular-weight chemicals that may be carried therein during the absorption process to high-molecular peptides, gene therapy drugs, and protein drugs. Moreover, the method has the potential to be used in various application fields as the method may be used in various formulations such as films, sponges, and patches as well as particles.

### BRIEF DESCRIPTION OF THE DRAWINGS

FIG. 1 is a diagram for illustrating a method for preparing a swellable polymer having an object immobilized thereto according to the present disclosure. a in FIG. 1 shows a conventional preparing method and b in FIG. 1 shows a preparing method according to the present disclosure.
FIG. 2 is a diagram for comparing immobilization efficiencies of an object ¹³¹I of Present Example 1 as the preparing method according to the present disclosure and Comparative Example 1 as the conventional preparing method with each other.
FIG. 3 is a diagram showing the immobilization efficiency of an object (¹³¹I) based on a concentration of an oxidizing agent and an absorption time in the preparing method according to the present disclosure.
FIGS. 4 to 9 are diagrams for illustrating one embodiment of bonding of a linker and an object in a preparing method according to the present disclosure.

### DETAILED DESCRIPTIONS

Hereinafter, embodiments of the present disclosure will be described in detail with reference to the accompanying drawings. The present disclosure may be subjected to various changes and may have various forms. Thus, particular embodiments will be illustrated in the drawings and will be described in detail herein. However, this is not intended to limit the present disclosure to a specific disclosed form. It should be understood that the present disclosure includes all modifications, equivalents, and replacements included in the spirit and technical scope of the present disclosure. While describing the drawings, similar reference numerals are used for similar components. In the accompanying drawings, the dimensions of the structures are shown to be exaggerated to make the clarity of the present disclosure.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "including", "include", and "including" when used in this specification, specify the presence of the stated features, integers, operations, elements, and/or components, but do not preclude the presence or addition of one or more other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the present disclosure describes in detail a method for preparing a swellable polymer having an object immobilized thereto according to the present disclosure with referring to the drawings of the present disclosure.

FIG. 1 is a diagram for illustrating a method for preparing a swellable polymer having an object immobilized thereto according to the present disclosure. a in FIG. 1 shows a conventional preparing method and b in FIG. 1 shows a preparing method according to the present disclosure.

Referring to a in FIG. 1, the conventional method for preparing a polymer having an object immobilized thereto includes reacting the object and a linker that has a functional group that can bind to the object with a polymer and bonding the object and the linker to the polymer, and crosslinking the polymer. The conventional preparation method has the disadvantage of being difficult to use the method on-site because the method requires multiple steps and a long time, which may reduce the activity of the object. To solve this problem, a method for reducing the reaction time by increasing a concentration of the object is generally used. However, in this approach, the object does not dissolve at a certain concentration thereof that exceeds solubility thereof. In addition, in the conventional method, the object binds to the polymer at very low efficiency.

On the contrary, referring to b in FIG. 1, the preparing method according to the present disclosure includes first reacting and binding the linker with the functional group that can bind to the object with and to the swellable polymer, and then drying the polymer, and absorbing an object solution dissolved or dispersed in a solvent that has affinity to the swellable polymer into the dried swellable polymer. In the method for preparing the swellable polymer in accordance with the present disclosure, a local concentration in the swellable polymer particle may be relatively increased via the absorption step so that the object can bind to a reactive group (linker) in the swellable polymer particle. Thus, the object can bind to the linker in a short time. Moreover, as long as the dried swellable polymer containing the linker with a functional group capable of binding to the object and the object solution dissolved or dispersed in the solvent that has affinity to the swellable polymer are available, the swellable polymer having the object immobilized thereto may be easily prepared on site at a very high binding ratio in a short period of time, which may also increase process efficiency.

Hereinafter, the present disclosure describes in more detail the method for preparing the swellable polymer having the object immobilized thereto according to the present disclosure based on specific examples and comparative examples. However, the examples of the present disclosure are only some embodiments of the present disclosure, and the scope of the present disclosure is not limited to the following examples.

### [Present Example 1]

To synthesize hyaluronic acid methacrylate (HAMA) used in preparing a swellable polymer, hyaluronic acid (1 g) was added to deionized water (10 mL) at 25°C, and was dissolved therein for 1 hour at 1000 RPM (rotation per minute) using a stirrer to prepare a solution. Then, a temperature of the solution was lowered to 4°C. While methacrylic anhydride (1.6g) was added thereto, pH of the solution was adjusted to 9 using 1M NaOH. Reaction thereof was carried out for 24 hours to prepare a mixture. To remove unreacted substances from the mixture, the mixture was put on a cellulose membrane (Molecular weight cut-off: 3500), and dialysis was performed in deionized water for 96 hours while replacing the deionized water every 8 hours. The dialyzed product was lyophilized to obtain cross-linkable HAMA. Next, a reaction was performed to introduce a reactive group (linker, API) that binds to the object (radioactive iodine, ¹³¹I) into HAMA. For this purpose, HAMA polymer (1 g) was added to phosphate-buffered saline (PBS) (100 mL) with a pH of 7.4, and dissolved therein at 1000 RPM for 1 hour using a stirrer to prepare a polymer solution. Then, 1-ethyl-3-(3-dimethylaminopropyl)carbodiimide (EDC) (900 mg) and N-hydroxysuccinimide (1100 mg) was added to the polymer solution, followed by stirring at 1000 RPM for 1 hour. Then, a solution in which API (600 mg) was dissolved in 40 mL of PBS was added to the mixture, followed by stirring for 24 hours to obtain a reaction product. To remove unreacted substances from the reaction product, the product was put on a cellulose membrane (Molecular weight cut-off: 3500), and dialysis was performed in deionized water for 96 hours while replacing the deionized water every 8 hours. The dialyzed product was lyophilized to obtain HAMA-API in which API bound to HMMA.

Next, in order to prepare a swellable polymer that may fix the object thereto, the HAMA-API (50 mg) and lithium phenyl-2,4,6-trimethylbenzoylphosphinate (LAP) (1 mg) as photoinitiator were dissolved in deionized water (1 mL), and the mixed solution was dispersed into droplets of a size of several tens of microns on oil, and 365 nm light with an intensity of 300 mW/cm² was irradiated thereto for 60 seconds such that cross-linked polymer in a form of hydrogel particles was prepared. To remove impurities (the oil, the photoinitiator, etc.) therefrom, the crosslinked polymer was washed twice with ethyl alcohol and deionized water, and the crosslinked polymer particles dispersed in the deionized water were freeze-dried to produce the swellable polymer that may fix the object (¹³¹I) thereto. Na¹³¹I (100 µL) aqueous solution containing chloramine T (0.5 mg) at a concentration of 1 mCi was added to a vial containing the dried swellable polymer in a form of particles, followed by vortexing for 10 minutes. Then, sodium metabisulfite solution (10 mg/mL, 10 µL) was added thereto to quench labeling of the radioactive iodine. The supernatant was removed therefrom and a resulting product was washed with additional ethanol and deionized water to prepare the swollen polymer having the radioactive iodine immobilized thereto.

### Comparative Example 1

Comparative Example 1 of the present disclosure was prepared using the conventional method. A first solution was prepared by dissolving HA (240 mg) in deionized water (20 mL) at 25°C. pH of the first solution was adjusted to pH 9 using 1M NaOH at 4°C, and then methacrylic anhydride (420 mg) was added thereto, followed by stirring for 24 hours. Thus, a mixture was prepared. The mixture was dialyzed in deionized water every 8 hours for 96 hours, and then the dialyzed product was lyophilized to obtain hyaluronic acid methacrylate (HAMA). Then, the HAMA reacted with N-(3-Aminopropyl)-imidazole, such that the API bound to HAMA via an amidation reaction between a carboxyl group of HAMA and an amine group of API. Specifically, HAMA (240 mg) was dissolved in phosphate-buffered saline (PBS) (40 mL, pH 7.4) at 25°C for 4 hours, and EDC (230 mg, 1.2 mmol) and NHS (276 mg, 2.4 mmol) were added thereto, followed by stirring for 30 minutes to activate the carboxyl group of HAMA. Then, a solution in which API (150 mg, 1.2 mmol) was dissolved in PBS (10 mL) was added thereto in a dropwise manner and reaction thereof was performed for 12 hours. The resulting product was dialyzed using PBS and freeze-dried to obtain API-conjugated HAMA (HAMA-API).

Next, in order to fix iodine as the object to HAMA-API, chloramine T was used as a catalyst (oxidizing agent) for iodination of an imidazole ring of HAMA-API. HAMA-API (24 mg) was dissolved in 500 µL PBS (pH 7.4) in a 1.5 mL centrifugal separation tube, and 100 µL Na¹³¹I was added thereto for 10 minutes. Then, a chloramine T solution (5 mg/mL, 10 µL) in which the chloramine T was dissolved in PBS (pH 7.4) was added to the mixture, followed by vortexing for 10 minutes. The iodination was quenched by adding sodium metabisulfite solution (10 mg/mL, 10 µL) thereto. To remove residues therefrom, ethyl alcohol (1 mL) was added thereto, and the mixture was centrifuged at 5000 rpm for 20 minutes. The supernatant was removed therefrom, and iodine-labeled HAMA (I-HAMA or ¹³¹I-HAMA) was obtained.

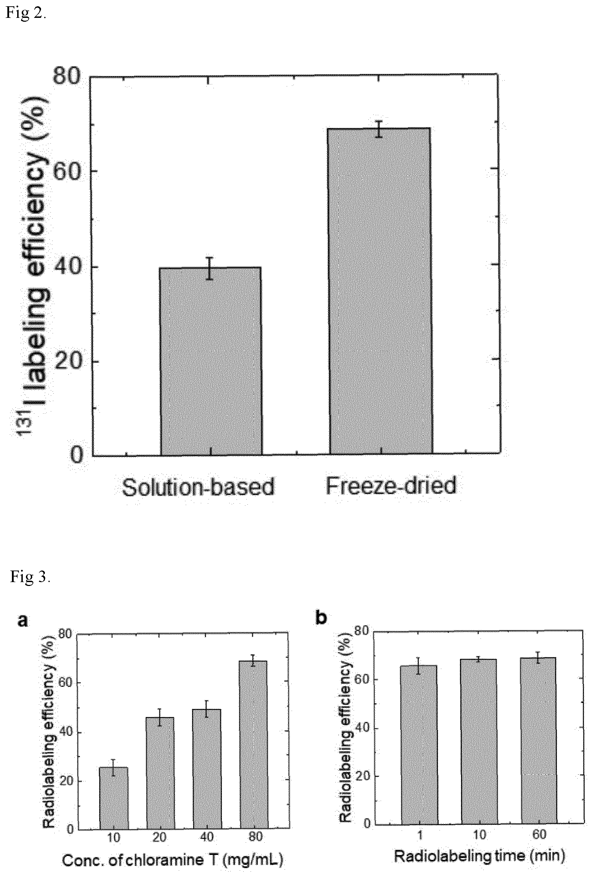

FIG. 2 is a diagram for comparing immobilization efficiencies of an object ¹³¹I of Present Example 1 as the preparing method according to the present disclosure and Comparative Example 1 as the conventional preparing method with each other.

Referring to FIG. 2, it may be identified that when using the conventional solution-based preparing method, the immobilization efficiency of ¹³¹I as the object is very low at about 39%, whereas when using the preparing method of the present disclosure using the swellable polymer (Freeze-dried), the immobilization efficiency of ¹³¹I is about 68%, thereby providing significantly higher object immobilization efficiency compared to the prior art.

In FIG. 3, a shows the immobilization efficiency of ¹³¹I based on the concentration of chloramine T as the oxidizing agent, and b) shows the immobilization efficiency of ¹³¹I based on a absorption reaction time of a dried HAMA-API swellable polymer and a ¹³¹I solution.

Referring to a in FIG. 3, it may be identified that the immobilization efficiency of ¹³¹I increases as the concentration of the oxidizing agent increases. Referring to b in FIG. 3, it may be identified that the ¹³¹I solution is absorbed into the HAMA-API swellable polymer, 65% or larger of ¹³¹I is immobilized thereto in one minute. Therefore, it may be identified that the preparing method according to the present disclosure is a method that may very quickly fix the object to the swellable polymer by simply reacting the solution containing the object with the dried swellable polymer. Thus, the decline in object activity may be minimized. It may be expected that the swellable polymer having the object immobilized thereto may be prepared easily and quickly at any site.

### [Present Example 2]

### HAMA swellable polymer having immobilized cationic metal thereto

A reaction was performed to introduce the chelator (linker, DTPA) that binds to an object (cationic radioisotope) into HAMA. For this purpose, HAMA polymer (0.7g) was added to phosphate-buffered saline (PBS) (30mL) with a pH of 7.4 and dissolved therein at 1000 RPM for 1 hour using a stirrer to prepare a polymer solution. Afterwards, 4-(4,6-dimethoxy-1,3,5-triazin-2-yl)-4-methylmorpholinium chloride (DMTMM) (0.35 g) was added to the polymer solution, followed by stirring at 1000 RPM for 6 hours. Then, a solution in which p-NH2-Bn-DTPA (0.42 g) was dissolved in 2 mL of PBS was added to the mixture, followed by stirring for 18 hours to obtain a reaction product. In order to remove unreacted substances from the product, the product was filled into a cellulose membrane and dialysis was performed thereon in deionized water for 72 hours while replacing the deionized water every 12 hours. The dialyzed product was lyophilized to obtain HAMA-DTPA in which DTPA bound to HAMA.

Next, in order to prepare the swellable polymer that may fix the object thereto, H0AMA-DTPA(50 mg) and LAP (10 mg) as a photoinitiator were dissolved in deionized water (1 mL) and then was dispersed into droplets of a size of several tens of microns on the oil. After dispersion, 365 nm light with an intensity of 7 W/cm2 was irradiated thereto for 60 seconds to prepare a cross-linked polymer in the form of hydrogel particles. Thereafter, to remove impurities (the oil, the photoinitiator, etc.), the crosslinked polymer was washed three times with ethyl alcohol and deionized water, and the crosslinked polymer particles dispersed in the deionized water were subjected to a freeze-drying process. Thus, a swellable polymer capable of immobilizing the object (cationic radioactive isotope) thereto was obtained. An aqueous solution of ZrCl₄ (100 µL) at a 5 to 6 mCi concentration was added to a vial containing the dried swellable polymer (3 mg) in the form of particles, and the reaction thereof was carried out for 1 hour with a stirrer at 27°C and 15,000 RPM. The supernatant was removed therefrom and the resulting product was washed with additional deionized water to prepare the swollen polymer to which radioactive zirconium was immobilized.

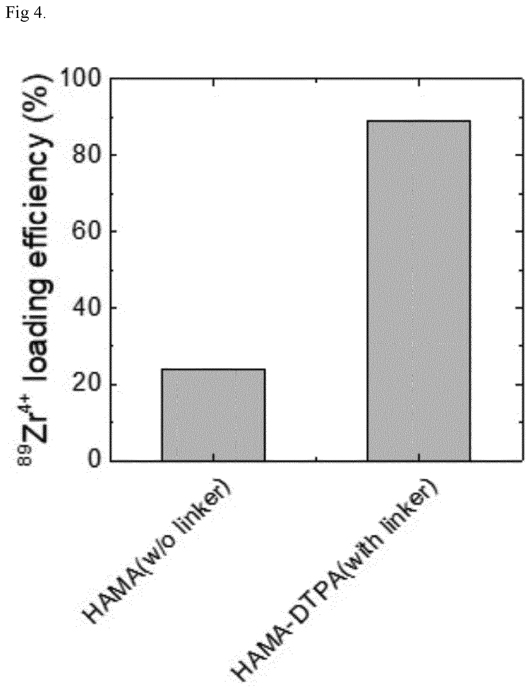

According to FIG. 4, the immobilization efficiency at which the swellable polymer fixes zirconium ions thereto depending on a type of the swellable polymer may be identified. It was identified that a DTPA-conjugated swellable polymer capable of immobilizing the cationic metal thereto exhibited a immobilization efficiency of approximately 90%.

### [Present Example 3]

### HAMA swellable polymer having immobilized doxorubicin thereto

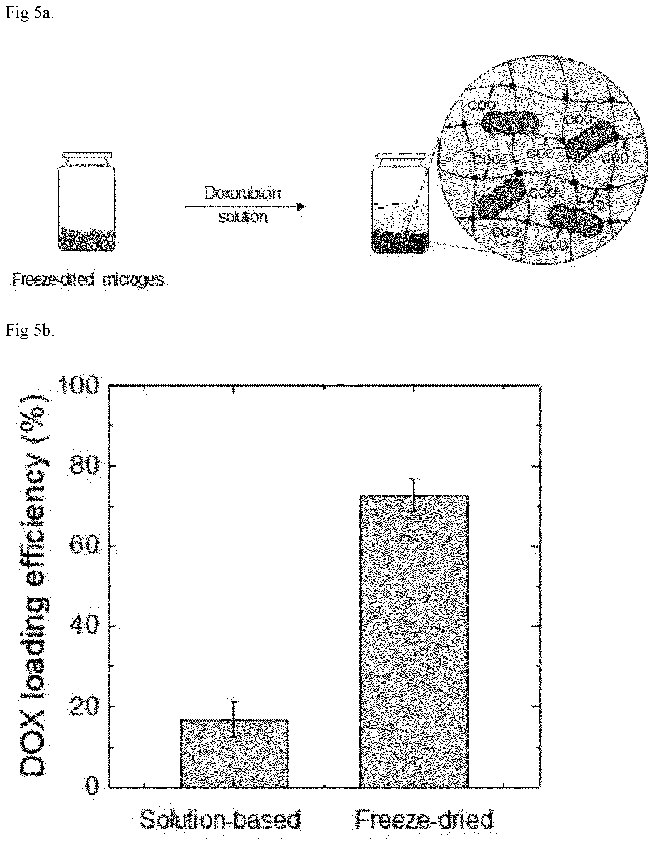

FIG. 5A is a diagram showing a preparing process of Present Example 3 according to the preparing method according to the present disclosure.

Referring to FIG. 5A, Present Example 3 of the present disclosure refers to a method of immobilizing the object with a cationic amine group to the swellable polymer with an anionic carboxyl group via ionic bonding. In each of a container containing hyaluronic acid swellable polymer swollen in deionized water, and a container containing the freeze-dried hyaluronic acid swellable polymer, 1 mL (5 mg/mL) of doxorubicin solution as the immobilizing target with an amine group was added thereto and was absorbed into the polymer. In the absorption process, the solvent was adjusted to pH (5 to 5.5) at which the two substances can be ionized. The reaction was carried out for 1 hour with a stirrer.

After the reaction, the swellable polymer was precipitated at 3000 RPM for 3 minutes using a centrifugal separator, and the supernatant that did not participate in the reaction was obtained therefrom and the absorbance at 490 nm thereof was analyzed using a UV spectrometer. According to FIG. 5B, it may be identified that, compared to the solution-based preparing method, in the freeze-drying based method, the doxorubicin may be immobilized to the swellable polymer at a loading efficiency (LE) of about 70% or greater.

### [Present Example 4]

### HAMA swellable polymer having immobilized cationic drug thereto

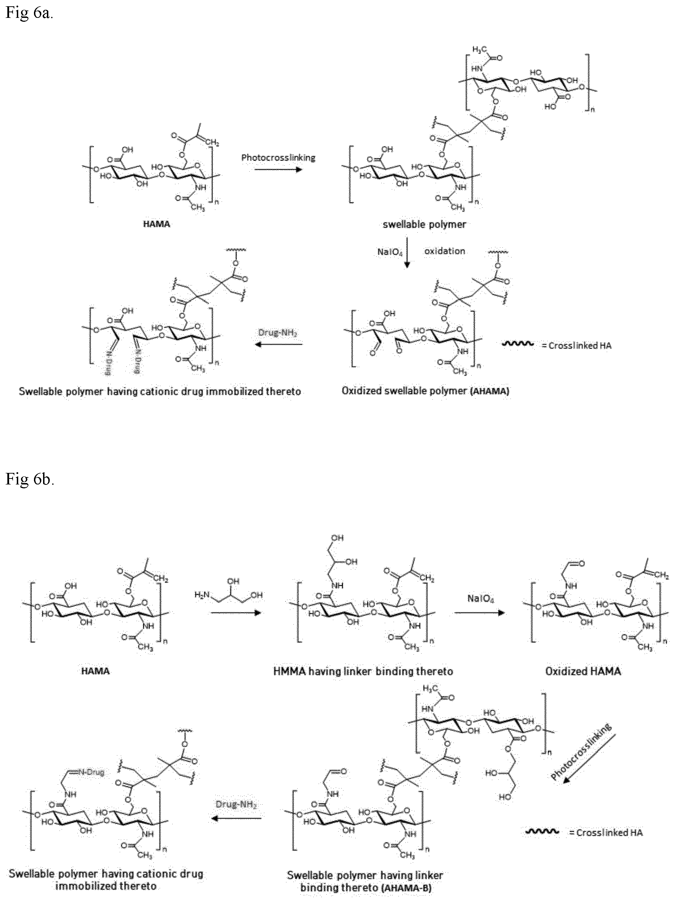

FIG. 6A and FIG. 6B are diagrams showing a preparing process of Present Example 4 according to the preparing method according to the present disclosure.

Referring to FIG. 6A, Present Example 4 of the present disclosure was performed based on a bond between an immobilization target having an amine group and a linker including an aldehyde group. First, in order to provide a linker containing an aldehyde group to the hyaluronic acid methacrylate (HAMA) swellable polymer to which a methacrylate group containing an aldehyde group is bound, HAMA was reacted with sodium periodate (NaI0₄) for 24 hours, and, then, cross-linking and freeze-drying were performed thereon. Thus, the dried HAMA-aldehyde swellable polymer was prepared. Then, the dried HAMA-aldehyde swellable polymer was reacted with 5-aminofluorescein as an immobilization target having an amine group. During the absorption process, the amine group of 5-aminofluorescein reacted with the aldehyde group of the HAMA-aldehyde swellable polymer, such that the HAMA swellable polymer to which the cationic drug was bound was prepared.

Moreover, in FIG. 6B, in order to provide the linker containing an aldehyde group to the HAMA swellable polymer, 3-amino-1,2-propanediol was reacted with HAMA, followed by crosslinking and freeze-drying. Thus, the swellable polymer to which the linker was bound was prepared. Thereafter, the swellable polymer to which the linker was bound reacted with sodium periodate (NaI0₄), followed by freeze-drying, such that the dried HAMA-aldehyde swellable polymer was prepared. The step of absorbing the 5-aminofluorescein solution as an immobilization target having an amine group into the dried HAMA-aldehyde swellable polymer was performed such that the amine group of 5-aminofluorescein was bound to the aldehyde group of the dried HAMA-aldehyde swellable polymer. Therefore, the HAMA swellable polymer to which the cationic drug was immobilized via the bond between the amine group and the aldehyde group was prepared.

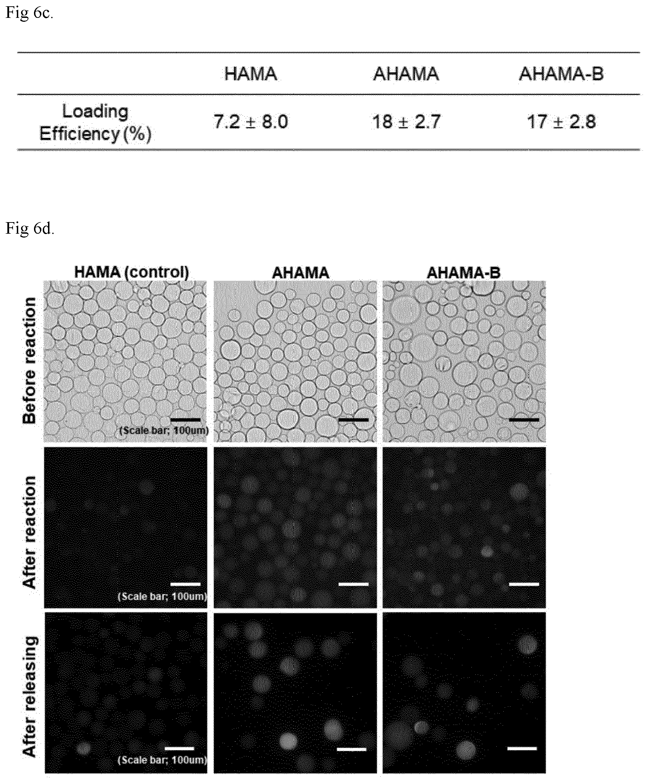

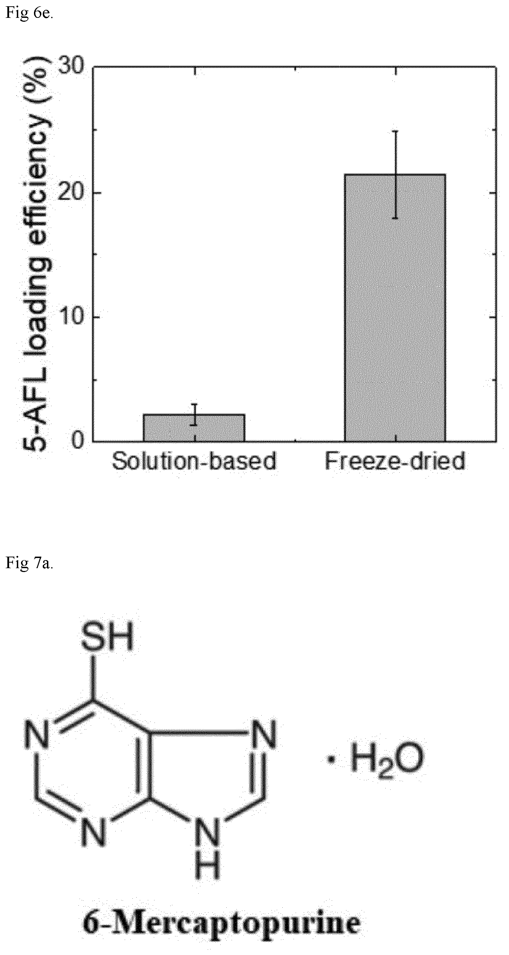

After the reaction, the swellable polymer was precipitated at 3000 RPM for 3 minutes using a centrifugal separator, and the supernatant that did not participate in the reaction was acquired, and the fluorescence at 520 nm thereof was analyzed using a fluorescence plate reader. According to FIG. 6C and FIG. 6D, it was identified that 5-aminofluorescein was immobilized to the swellable polymer at a loading efficiency (LE) of about 20%, compared to HAMA with no linker labeled thereto. FIG. 6E a diagram showing comparing results between a 5-AFL immobilization efficiency of Comparative Example 4 and a 5-AFL immobilization efficiency of Present Example 4 according to the preparing method according to the present disclosure. Referring to FIG. 6E, it may be identified that when using the conventional solution-based preparing method, the immobilization efficiency of 5-AFL which is an object is very low at about 2%, whereas when using the preparing method of the present disclosure using the swellable polymer (Freeze-dried), the immobilization efficiency of 5-AFL is about 21%, indicating that the preparing method of the present disclosure providing significantly higher object immobilization efficiency compared to the conventional technology.

### [Present Example 5]

### HAMA swellable polymer having immobilized mercaptopurine (6-Mercaptopurine) thereto

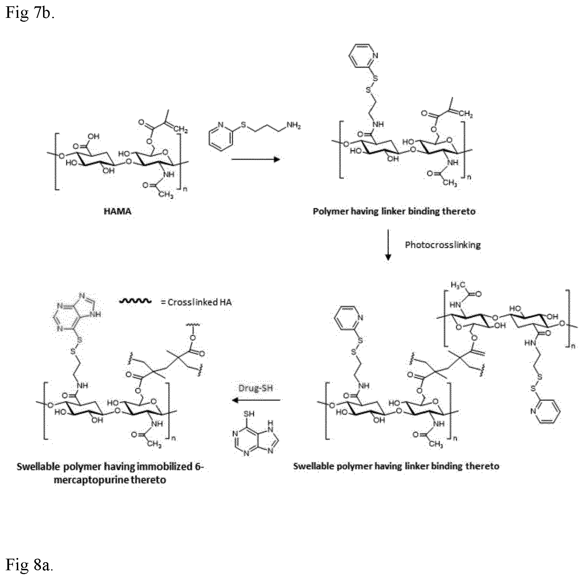

FIG. 7 is a diagram showing a preparing process of Present Example 5 according to preparing method according to the present disclosure.

Referring to FIG. 7, Present Example 5 of the present disclosure was performed based on a bond between an immobilization target having a disulfide (-S-S-, disulfide group) and a linker including a thiol group (thiol, -SH). To bind the linker containing the thiol group to the HAMA swellable polymer, HAMA swellable polymer reacted with (2-pyridylthio)cysteamine hydrochloride, followed by cross-linking and freeze-drying. Thus, the dried HAMA-thiol swellable polymer was prepared. Then, a solution containing mercaptopurine as a drug containing a disulfide group was absorbed into the dried HAMA-thiol swellable polymer. In the absorption process, the disulfide group of mercaptopurine and the thiol group of HAMA-thiol swellable polymer were bounded to each other, such that the HAMA swellable polymer to which mercaptopurine was immobilized was prepared.

### [Present Example 6]

### HAMA swellable polymer having immobilized chlorambucil thereto

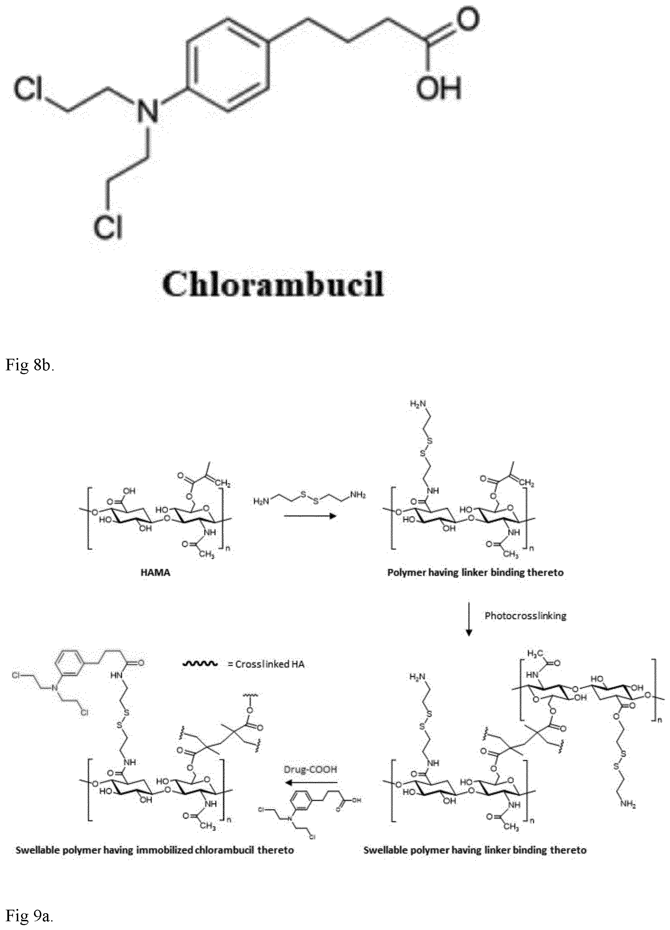

FIG. 8 is a diagram showing a preparation process of the HAMA swellable polymer to which chlorambucil is immobilized, according to the preparing method according to the present disclosure.

Referring to FIG. 8, Present Example 6 of the present disclosure was performed based on a bond between an object with a carboxyl group and a linker including an amine group. First, in order to provide a linker containing an amine group (-NH₂) to the HAMA polymer, cystamine was reacted with the HAMA polymer, followed by cross-linking and freeze-drying to prepare the swellable polymer to which the linker was bound. Then, a solution containing chlorambucil as a drug containing a carboxyl group was absorbed into the swellable polymer to which the linker was bound. In the absorption process, the carboxyl group of chlorambucil was bound to the amine group of the swellable polymer, such that the swellable polymer to which chlorambucil was bound was prepared.

### [Present Example 7]

### HAMA swellable polymer having immobilized paclitaxel thereto

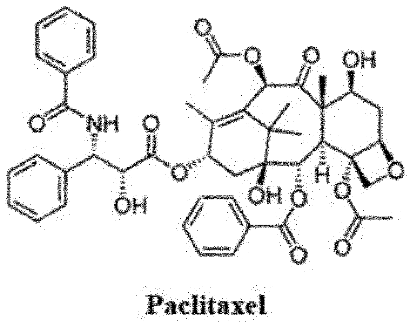

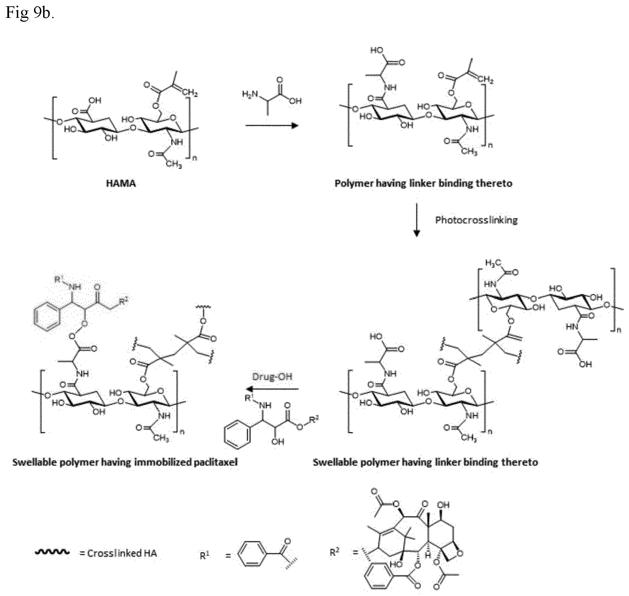

FIG. 9 is a diagram showing a preparing process of the HAMA swellable polymer to which paclitaxel is immobilized, according to the preparing method according to the present disclosure.

Referring to FIG. 9, Present Example 7 of the present disclosure was performed based on a bond between an object with a hydroxyl group and a linker including a carboxyl group. First, in order to provide a linker containing a carboxyl group to the HAMA polymer, alanine was reacted with the HAMA polymer, followed by cross-linking and freeze-drying to prepare a swellable polymer to which the linker was bonded. Then, a solution containing paclitaxel as a drug containing a hydroxyl group (-OH) was absorbed into the swellable polymer to which the linker was attached. During the absorption process, the hydroxyl group of paclitaxel and the carboxyl group of the swellable polymer were bound to each other, such that the swellable polymer to which paclitaxel was immobilized was prepared.

As shown in the Present Examples, the dried swellable polymer containing the linker with the functional group capable of bonding to the object in the preparing method according to the present disclosure may be prepared by bonding the linker to the polymer, followed by crosslinking and dry-freezing thereof, or may be prepared by crosslinking and drying the polymer, and then, bonding the linker to the polymer.

Although the description is made above with reference to preferred embodiments of the present disclosure, those skilled in the art may modify and change the present disclosure in various ways without departing from the spirit and scope of the present disclosure as set forth in the patent claims below.

Abbreviations used herein are as follows.
DOTA
1,4,7,10-tetraazacyclododecane-1,4,7,10-tetraacetic acid
DOTA-GA
2-(4,7,10-tris(carboxymethyl)-1,4,7,10-tetraazacyclododecan-1-yl)pentanedioic acid
DO2A
1,4,7,10-tetraazacyclododecane-1,7-diacetic acid
HBED-CC
3,3'-(3,3'-(ethane-1,2-diylbis((carboxymethyl)azanediyl))bis(methylene) bis(4-hydroxy-3,1-phenylene))dipropanoic acid
NOTA
1,4,7-triazonane-1,4,7-triacetic acid
p-SCN-Bn-NOTA
2-(4-isothiocyanatobenzyl)-NOTA
NODA-GA
2-(4,7-bis(carboxymethyl)-1,4,7-triazonan-1-yl)pentanedioic acid
PrP9
1,4,7-triazonane-1,4,7-tri(2-carboxyethyl)methyl phosphinic acid
DTPA
Diethylenetriamine-pentaacetic acid
p-SCN-CHX-A"-DTPA
2S-(4-isothiocyanatobenzyl)cyclohexyldiethylenetriamine-pentaacetic acid
p-SCN-CHX-B"-DTPA
2R-(4-isothiocyanatobenzyl)cyclohexyldiethylenetriamine-pentaacetic acid
NETA
(2-(4,7-bis(carboxymethyl)-1,4,7-triazonan-1-yl)ethylazane-diacetic acid
NE3TA
7-(2-(carboxymethylamino)ethyl)-1,4,7-triazonane-1,4-diacetic acid
NE3TA-Bn
(7-(2-(benzyl(carboxymethyl)amino)ethyl)-1,4,7-triazonane-1,4-diacetic acid
C-NETA
2,2'-(2-(4,7-bis(carboxymethyl)-1,4,7-triazonan-1-yl)-(2-(4-isothiocyanatobenzyl)) ethylazanediyl)diacetic acid
C-NE3TA
2,2'-(7-(2-(carboxymethylamino)-2-(4-isothiocyanatobenzyl)ethyl)- 1,4,7-triazonane-1,4-diyl)diacetic acid
C-DOTA
2-(4-nitrobenzyl)-DOTA
1B3M-DTPA
1-benzyl-3-methyldiethylenetriamine-pentaacetic acid
1B4M-DTPA
1-benzyl-4-methyldiethylenetriamine-pentaacetic acid
1M3B-DTPA
1-methyl-3-benzyldiethylenetriamine-pentaacetic acid
TETA
1,4,8,11-tetraaza-cyclotetradecane-1,4,8,11-tetraacetic acid
TE2A
1,4,8,11-tetraazacyclo-tetradecane-1,8-diacetic acid
CB-TE2A
1,4,8,11-tetraazabicyclo[6.6.2]hexadecane-4,11-diacetic acid
CB-DO2A
1,4,7,10-tetraazabicyclo[5.5,2]tetradecane-4,10-diacetic acid
DiAmSar
3,6,10,13,16,19-hexaazabicyclo[6.6.6]icosane-1,8-diamine
SarAr
N-(4-aminobenzyl)-3,6,10,13,16,19-hexaazabicyclo[6.6.6]icosane-1,8-diamine
AmBaSar
4-((8-amino-3,6,10,13,16,19-hexaazabicyclo[6.6.6]icosan-1-ylamino)methyl)benzoic acid
AAZTA
6-amino-6-methylperhydro-1,4-diazepinetetraacetic acid

## Claims

1. A method for preparing a swellable polymer having an object immobilized thereto, the method comprising:
absorbing an object solution into a dried swellable polymer, wherein the dried swellable polymer contains a linker having a functional group capable of binding to an object, wherein the object solution includes a solvent having affinity to the swellable polymer, and the object dissolved or dispersed in the solvent.

2. The method for preparing the swellable polymer having the obj ect immobilized thereto of claim 1, wherein the swellable polymer is a hydrogel, and the solvent is water.

3. The method for preparing the swellable polymer having the obj ect immobilized thereto of claim 1, wherein the swellable polymer is a cross-linked hydrophobic polymer,
wherein the solvent is an organic solvent.

4. The method for preparing the swellable polymer having the obj ect immobilized thereto of claim 1, wherein the drying is freeze-drying.

5. The method for preparing the swellable polymer having the obj ect immobilized thereto of claim 1, wherein the object is at least one a radioactive isotope, a metal, an ionic compound, and an organic compound.

6. The method for preparing the swellable polymer having the obj ect immobilized thereto of claim 1, wherein the object is a metal or an ionic compound,
wherein the linker is a ligand compound capable of having a coordinate bond to the metal or the ionic compound,

7. The method for preparing the swellable polymer having the obj ect immobilized thereto of claim 1, wherein the object has an anionic functional group,
wherein the functional group of the linker is an aromatic compound or a heterocyclic compound,
wherein an electrophilic substitution reaction between the anionic functional group and the functional group of the linker occurs.

8. The method for preparing the swellable polymer having the object immobilized thereto of claim 7, wherein the heterocyclic compound is selected from a group consisting of imidazole, pyrrole, furan, thiophene, indole, and 3,4-dihydroxyphenyl.

9. The method for preparing the swellable polymer having the obj ect immobilized thereto of claim 1, wherein the object has a cationic functional group, and the ligand is a chelator.

10. The method for preparing the swellable polymer having the obj ect immobilized thereto of claim 9, wherein the chelator is selected from a group consisting of DOTA, DOTA derivatives, DOTA-GA, DO2A, HBED-CC, NOTA, p-SCN-Bn-NOTA, NODA-GA, PrP9, DTPA, CHX-A"-DTPA-isothiocyanate, CHX-A"-DTPA-maleimide, CHX-A"-DTPA-glutaric acid NHS ester, DTPA derivatives (1M3B-DTPA, 1B3M-DTPA, 1B4M-DTPA, CHX-A-DTPA and CHX-B-DTPA), NETA, NE3TA, NE3TA-Bn, C-NETA, C-NE3TA, C-DOTA-isothiocyanates, MeO-DOTA-isothiocyanates, PA-DOTA-isothiocyanates, 1B4M-DTPA-isothiocyanates, 2-(4-nitrobenzyl)-cyclen, 2-(4-nitrobenzyl)-cyclam, 2-(4-benzamidobenzyl)-NOTA, 2-(4-nitrobenzyl)-DOTA, 2-(4-nitrobenzyl)-TETA, 1-(4-nitro)-B4MDTPA, TETA, TE2A, TE2A derivatives (CB-TE2A, CB-TE1A1P, CB-TE2P, MM-TE2A, DM-TE2A), cyclam, cyclam derivatives (1-(4-nitrobenzyl)-cyclam, 6-(4-nitrobenzyl)-cyclam), CB-TE2A, CB-TE2A derivatives (3,3'-(1,4,8,11-tetraazabicyclo[6.6.2]hexadecane-4,11-diyl)dipropanoic acid), CB-DO2A, CB-DO2A derivatives (3,3'-(1,4,7,10-tetraazabicyclo[5.5.2]tetradecane-4,10-diyl)dipropanoic acid), DiAmSar, SarAr, AmBaSar, DiAmsar derivatives, AAZTA, NOPO and TACN-TM.

11. The method for preparing the swellable polymer having the obj ect immobilized thereto of claim 1, wherein the object is an organic compound,
wherein the organic compound includes one of an amine group, a carboxyl group, a hydroxyl group, and a thiol group,
wherein the linker has the functional group capable of binding to a functional group of the organic compound.

12. The method for preparing the swellable polymer having the object immobilized thereto of claim 11, wherein the functional group of the organic compound is an amine group,
wherein the functional group of the linker is an aldehyde group.

13. The method for preparing the swellable polymer having the obj ect immobilized thereto of claim 11, wherein the functional group of the organic compound is a carboxyl group,
wherein the functional group of the linker is an amine group.

14. The method for preparing the swellable polymer having the object immobilized thereto of claim 11, wherein the functional group of the organic compound is a hydroxyl group,
wherein the functional group of the linker is a carboxyl group.

15. The method for preparing the swellable polymer having the obj ect immobilized thereto of claim 11, wherein the functional group of the organic compound is a thiol group,
wherein the functional group of the linker is a disulfide group.
